# EUROPEAN PATENT SPECIFICATION

(11) **EP 4 049 666 B1**
(45) Date of publication and mention of the grant of the patent: **30.10.2024**
(21) Application number: 20884431.6
(22) Date of filing: 09.11.2020
(51) Int. Cl.: A61K 35/34, A61L 27/36, A61L 27/38, A61P 9/10, C12N 5/077, A61K 9/00

(54) **CELL CULTURE FOR TREATING PERIPHERAL ARTERIAL DISEASE**
ZELLKULTUR ZUR BEHANDLUNG DER PERIPHEREN ARTERIELLEN VERSCHLUSSKRANKHEIT
CULTURE CELLULAIRE POUR LE TRAITEMENT DE L' ARTERIOPATHIE OBLITERANTE DE L'AORTE ET MEMBRES INFERIEURS

(30) Priority: 08.11.2019 JP 2019203477
(43) Date of publication of application: 31.08.2022
(73) Proprietor: OSAKA UNIVERSITY, Suita-shi Osaka 565-0871 (JP); TERUMO Kabushiki Kaisha, Tokyo 151-0072 (JP)
(72) Inventor: SAWA, Yoshiki, Suita-shi, Osaka 565-0871 (JP); MIYAGAWA, Shigeru, Suita-shi, Osaka 565-0871 (JP); MIYAKE, Keisuke, Suita-shi, Osaka 565-0871 (JP); OYAMA, Kenji, Ashigarakami-gun, Kanagawa 259-0151 (JP)
(74) Representative: Prüfer & Partner mbB Patentanwälte · Rechtsanwälte
(86) International application number: PCT/JP2020/041662
(87) International publication number: WO 2021/090945

(56) References cited:
- JP-A- 2018 521 018
- JP-A- 2018 521 018
- HUANG CHIEH-CHENG ET AL: "Injectable Cell Constructs Fabricated via Culture on a Thermoresponsive Methylcellulose Hydrogel System for the Treatment of Ischemic Diseases", ADVANCED HEALTHCARE MATERIALS, vol. 3, no. 8, 27 January 2014 (2014-01-27), DE, pages 1133 - 1148, XP055821882, ISSN: 2192-2640, DOI: 10.1002/adhm.201300605
- KIM DONG WAN ET AL: "Therapeutic angiogenesis by a myoblast layer harvested by tissue transfer printing from cell-adhesive, thermosensitive hydrogels", BIOMATERIALS, vol. 34, no. 33, 1 November 2013 (2013-11-01), AMSTERDAM, NL, pages 8258 - 8268, XP055821883, ISSN: 0142-9612, DOI: 10.1016/j.biomaterials.2013.07.071
- HUANG CHIEH-CHENG, LIAO ZI-XIAN, CHEN DING-YUAN, HSIAO CHUN-WEN, CHANG YEN, SUNG HSING-WEN: "Injectable Cell Constructs Fabricated via Culture on a Thermoresponsive Methylcellulose Hydrogel System for the Treatment of Ischemic Diseases", ADV. HEALTHCARE MATER., vol. 3, no. 8, 2014, pages 1133 - 1148, XP055821882
- KIM DONG WAN, JUN INDONG, LEE TAE-JIN, LEE JI HYE, LEE YOUNG JUN, JANG HYEON-KI, KANG SEOKYUNG, PARK KI DONG, CHO SEUNG-WOO, KIM B: "Therapeutic angiogenesis by a myoblast layer harvested by tissue transfer printing from cell -adhesive, thermosensitive hydrogels", BIOMATERIALS, vol. 34, no. 33, 2013, pages 8258 - 8268, XP055821883
- KEISUKE MIYAKE, SHIGERU MIYAGAWA, TAKASHI SHIBUYA, AKIMA HARADA, SHUSAKU MAEDA, TAKAYOSHI UENO, TORU KURATANI, KOICHI TODA AND YOS: "Clusetered Myoblast Cell Injection Utilizing Cells Sheet Technology Augment Angiogenesis and Muscle Regeneration in a Mouse Model of Peripheral Artery Disease", CIRCULATION, vol. 140, no. Suppl. 1, 11 November 2019 (2019-11-11), US , pages A10878, XP009536630, ISSN: 0009-7322, DOI: 10.1161/circ.140.suppl_1.10878

## Description

### Technical Field

The present invention relates to a fragment of a sheet-shaped cell culture, wherein the fragment has a size of 100 to 500 µm, and wherein the fragment comprises an extracellular matrix on an outer surface of the cell culture, for use in treating a disease in the lower limbs, wherein this disease is peripheral arterial disease; to a fragment of a sheet-shaped cell culture, comprising an extracellular matrix on an outer surface of the cell culture, wherein the fragment of the sheet-shaped cell culture has a size of 100 to 500 µm; to a method for producing the fragment of a sheet-shaped cell culture containing myoblasts; and to a pharmaceutical composition for use in a method of treating peripheral arterial disease comprising the fragment of a sheet-shaped cell culture, wherein the fragment of the cell culture has a size of 100 to 500 µm, and wherein the method comprises administering the pharmaceutical composition.

### Background Art

In recent years, with the progress of aging, the westernization of eating habits, the lack of exercise, and the like, the number of patients with arteriosclerosis has been increased, and further the number of patients with peripheral arterial disease has been also increased. As a method for treating such a disease, guidance on lifestyle modification, or drug therapy is selected when the disease is minor, and when the disease is severe such as critical limb ischemia, a therapeutic method such as an angiogenesis therapy using a catheter, or a bypass surgery is selected. However, if the disease becomes more severe, it becomes impossible to treat the disease even with such a therapeutic method, and amputation of the ischemic limb is required. For this reason, it is desired to develop a new therapeutic method that is less invasive and can be used for a patient with severe disease.

In this regard, with the discovery of vascular endothelial progenitor cells, the development of a therapeutic method utilizing regenerative medicine for such a disease has been promoted. Examples of the therapeutic method include a method for transplanting a mononuclear cell fraction of autologous bone marrow cells to the ischemic limb of a subject (Non Patent Literature 1), a method for administering peripheral blood mononuclear cells or mesenchymal stem cells to a subject with peripheral arterial disease (Patent Literatures 1 and 2), and a method for transplanting iPS cell-derived vascular progenitor cells that are formed into a sheet shape with gel to a subject with lower limb ischemia (Patent Literature 3). Also known are compositions and methods using an engineered cardiac fibroblast-derived three-dimensional extracellular matrix. The compositions can for example be used to treat cardiac disease or ischemic disease or injury (Patent Literature 4).

Further known are injectable cell constructs that are fabricated via culture on a thermoresponsive methylcellulose hydrogel system for the treatment of ischemic diseases (Non Patent Literature 2), and therapeutic angiogenesis by a myoblast layer harvested by tissue transfer printing from cell-adhesive, thermosensitive hydrogels (Non Patent Literature 3).

### Citation List

### Patent Literature

Patent Literature 1: JP 2012-512843 W
Patent Literature 2: JP 2015-524437 W
Patent Literature 3: WO 2013/069661
Patent Literature 4: JP 2018-521018

### Non Patent Literature

Non Patent Literature 1: Toru Yoshioka, etc., J Jpn Coll Angiol, 2005, 45: 161-165
Non Patent Literature 2: Chieh-Cheng Huang, etc., Adv.
Healthcare Mater., 2014, 3: 1133-1148
Non Patent Literature 3: Dong Wan Kim, etc., Biomaterials, 2013, 34: 8258-8268

### Summary of Invention

### Technical Problem

It is an object of the present invention to provide a cell culture having an extracellular matrix on an outer surface of the cell culture, a method for producing the cell culture, a method for treating peripheral arterial disease using the cell culture, and the like.

### Solution to Problem

In a method for administering a mononuclear cell fraction of autologous bone marrow cells, peripheral blood mononuclear cells, or mesenchymal stem cells to a subject with peripheral arterial disease, disclosed in Patent Literatures 1 and 2, each is administered in a single-cell form, and there remains a problem that the bioadhesiveness of administered cells at the administration site is insufficient.

The present inventors have found that by administering a cell culture containing myoblast cells and having an extracellular matrix on an outer surface of the cell culture, the high bioadhesiveness is secured in the body and the angiogenesis can be promoted, and as a result of further studies based on such a finding, the present inventors have completed the present invention.

That is, the present invention relates to the subject-matter indicated in the independent claims. Preferred embodiments are indicated in the dependent claims.

### Advantageous Effects of Invention

By administering the fragment of the sheet-shaped cell culture of the present invention, angiogenesis is promoted, and peripheral arterial disease can be treated. Further, the fragment of the sheet-shaped cell culture of the present invention can be engrafted for a long time at an administration site. Further, the fragment of the sheet-shaped cell culture of the present invention can be administered directly to an affected part of a subject, for example, by injection, and thus, even if the affected part of a subject is widespread, the fragment of the sheet-shaped cell culture can be divided and easily administered to multiple points. In more detail, by administering the fragment of the sheet-shaped cell culture of the present invention to an affected part, for example, centrally nucleated muscle fibers can be generated, or the blood flow can be improved.

### Brief Description of Drawings

Fig. 1 shows measurement results of cell purity by using fluorescence-activated cell sorting (FACS) of the cells used for preparing a sheet-shaped cell culture.
Fig. 2A shows a photograph of a prepared sheet-shaped cell culture.
Fig. 2B shows a photomicrograph of a cross section of a hematoxylin-eosin stained sheet-shaped cell culture.
Fig. 2C shows a photomicrograph of a cross section of an immunostained sheet-shaped cell culture.
Fig. 3 shows photomicrographs of a suspension of a sheet-shaped cell culture. Fig. 3A is a photograph of a suspension prepared by repeating a set of suction and discharge 5 times as suspension.
Fig. 3B shows an enlarged photomicrograph of the suspension of Fig. 3A.
Fig. 3C is an enlarged photomicrograph of a suspension prepared by repeating a set of suction and discharge 10 times as suspension.

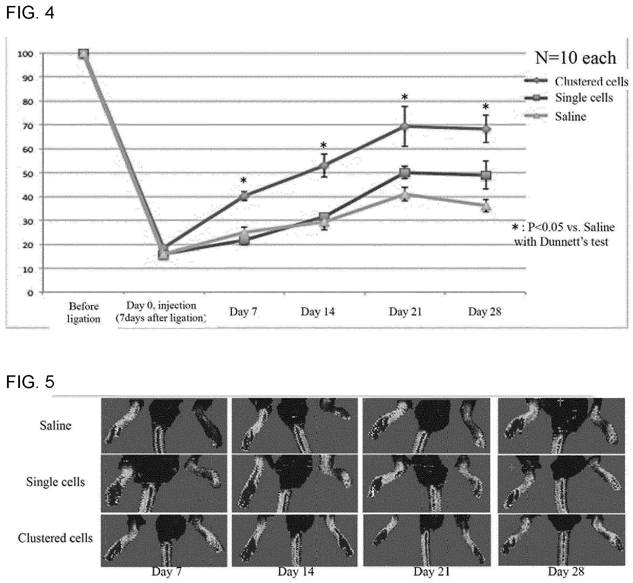

Fig. 4 is a graph showing results of blood flow % of a mouse in each group on days 0, 7, 14, 21, and 28 when the blood flow of the mouse before making into a mouse model of ischemia was set to 100%, as measured by laser Doppler perfusion imaging (LDPI). In the diagram, the expression "Clustered cells" indicates a mouse in the fragment administration group, the expression "Single cells" indicates a mouse in the single cell administration group, and the expression "Saline" indicates a mouse in the saline administration group. Further, in the diagram, the symbol "*" represents that Dunnett's test was performed on the saline administration group and P < 0.05 was obtained.
Fig. 5 shows imaging results of representative mice in respective groups on days 7, 14, 21 and 28 as measured by LDPI. In the diagram, the expression "Clustered cells" indicates a mouse in the fragment administration group, the expression "Single cells" indicates a mouse in the single cell administration group, and the expression "Saline" indicates a mouse in the saline administration group.

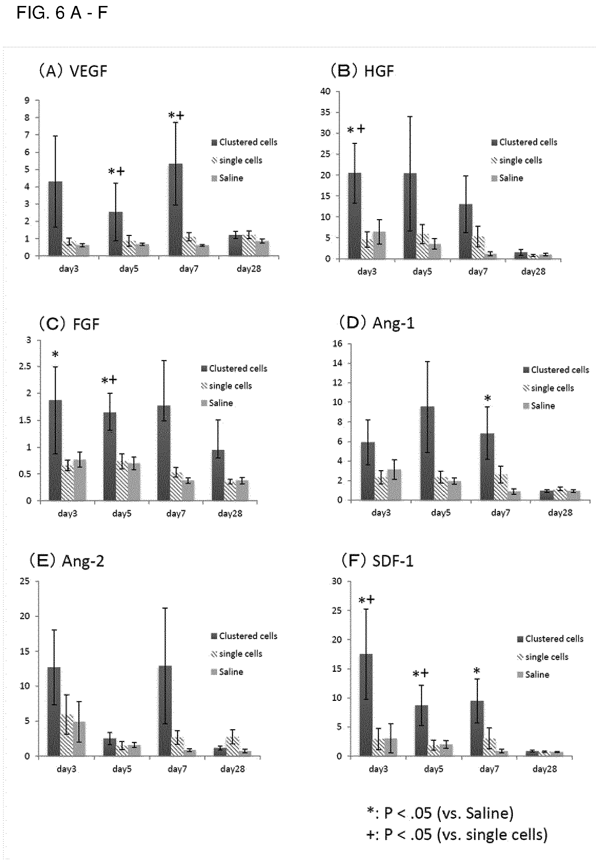

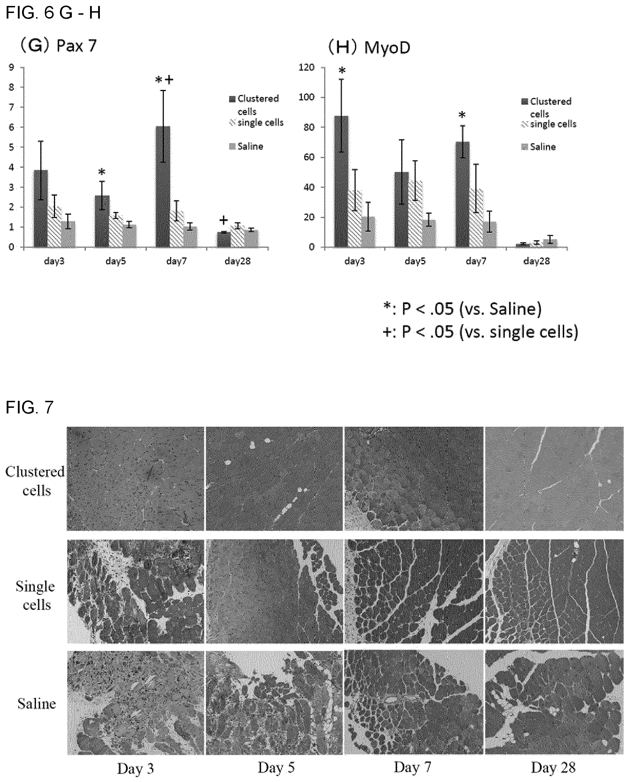

Fig. 6 shows results of RT-PCR in the tissues collected from respective mice in the fragment administration group, the single cell administration group, and the saline administration group, on days 3, 5, 7, and 28. In the diagram, the expression "Clustered cells" indicates a mouse in the fragment administration group, the expression "Single cells" indicates a mouse in the single cell administration group, and the expression "Saline" indicates a mouse in the saline administration group. Further, in the diagram, the symbol "*" represents that Dunnett's test was performed on the saline administration group and P < 0.05 was obtained, and the symbol "+" represents that Dunnett's test was performed on the single cell administration group and P < 0.05 was obtained. Fig. 6A shows results of RT-PCR for vascular endothelial growth factor (VEGF), Fig. 6B shows results of RT-PCR for hepatocyte growth factor (HGF), Fig. 6C shows results of RT-PCR for epidermal growth factor (FGF), Fig. 6D shows results of RT-PCR for angiopoietin-1 (Ang-1), Fig. 6E shows results of RT-PCR for angiopoietin-2 (Ang-2), and Fig. 6F shows results of RT-PCR for stromal cell-derived factor-1 (SDF-1).
Fig. 6G shows results of RT-PCR for Pax7, and Fig. 6H shows results of RT-PCR for MyoD.
Fig. 7 shows photomicrographs of the tissues collected from respective mice in the fragment administration group 2, the single cell administration group 2, and the saline administration group 2 and stained with hematoxylin and eosin, on days 3, 5, 7, and 28. In the diagram, the expression "Clustered cells" indicates a mouse in the fragment administration group 2, the expression "Single cells" indicates a mouse in the single cell administration group 2, and the expression "Saline" indicates a mouse in the saline administration group 2.

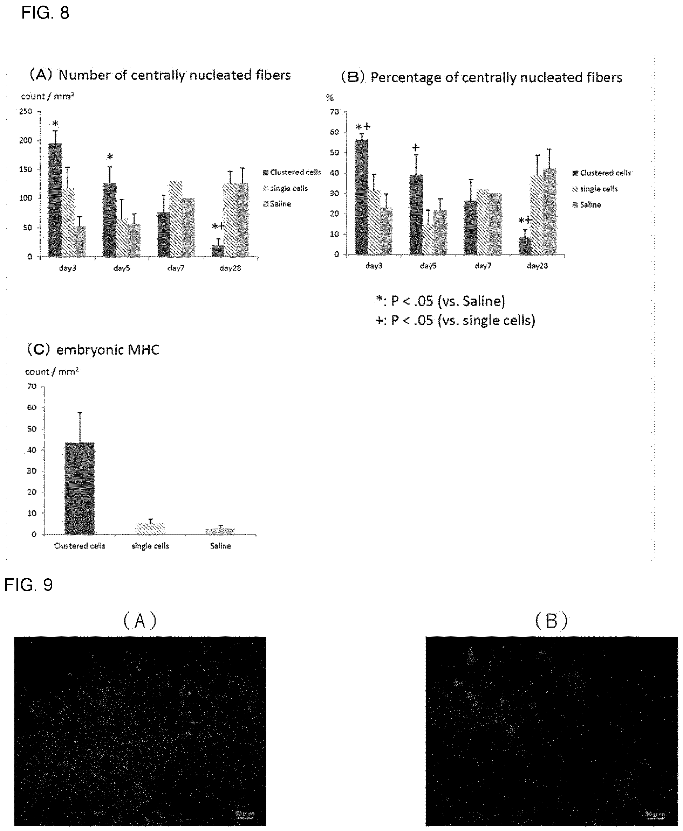

Fig. 8A shows results of counting the number of centrally nucleated muscle fibers (newly formed muscle fibers) in respective hematoxylin-eosin stained tissues. Fig. 8B shows results of calculating the ratio between the number of centrally nucleated muscle fibers (newly formed muscle fibers) and the number of mature muscle fibers having a nucleus in the margin (periphery) in respective hematoxylin-eosin stained tissues. In the diagram, the expression "Clustered cells" indicates a mouse in the fragment administration group 2, the expression "Single cells" indicates a mouse in the single cell administration group 2, and the expression "Saline" indicates a mouse in the saline administration group 2. Further, in the diagram, the symbol "*" represents that Dunnett's test was performed on the saline administration group 2 and P < 0.05 was obtained, and the symbol "+" represents that Dunnett's test was performed on the single cell administration group 2 and P < 0.05 was obtained. Fig. 8C shows results of counting the number of embryonic MHC-positive cells in the tissues collected from respective mice in the fragment administration group 2, the single cell administration group 2, and the saline administration group 2, on day 3. MHC is a marker of skeletal muscle, and when the marker is positive, it indicates that the cells are muscle cells.
Fig. 9 shows photomicrographs of frozen specimens of the tissues collected from respective mice in the fragment administration group 2 and the single cell administration group 2, on day 7. Fig. 9(A) shows a photograph of a specimen collected from a mouse in the fragment administration group 2, and Fig. 9(B) shows a photograph of a specimen collected from a mouse in the single cell administration group 2.

### Description of Embodiments

Hereinafter, the present invention will be described in detail.

One aspect of the present invention relates to a fragment of a sheet-shaped cell culture, wherein the fragment has a size of 100 to 500 µm, for use in treating disease in the lower limbs, having an extracellular matrix on an outer surface of the cell culture, wherein the disease in the lower limbs is peripheral arterial disease.

The expression "cell culture" in the present invention refers to a composition obtained through a cell culture step. In the fragment of a sheet-shaped cell culture for use of the present invention, at least part of the cells are joined to each other through an extracellular matrix produced by the cells forming the cell culture. The fragment of a sheet-shaped cell culture that is used in the present invention, has an extracellular matrix not only between the cells inside the cell culture, but also on an outer surface of the cell culture. In one embodiment, the fragment of the sheet-shaped cell culture for use in the present invention has a layer of extracellular matrix over the entire outer surface of the cell culture. The cell culture for use in the present invention has a sheet-shaped morphology. In this regard, the sheet-shaped morphology refers to a morphology having a first surface and a second surface that is substantially parallel to the first surface, and the cell culture has a layer of extracellular matrix on one surface thereof.

In the present invention, although depending on various conditions, the extracellular matrix produced by the cells forming a cell culture is generated, for example, by culturing the cells for 24 hours or more, for example, for 24 hours, 30 hours, 36 hours, 42 hours, 48 hours, 54 hours, 60 hours, 66 hours, or 72 hours, after seeding the cells.

The extracellular matrix contributes to the binding of a cell culture to an administration site, and can improve the survival rate of the cell culture at the administration site when the cell culture is administered. Further, the extracellular matrix is a cell-derived component, and thus, there is no problem in using the extracellular matrix for administration together with the cell culture that is used in the present invention. The fragment of the sheet-shaped cell culture for use in the present invention has an extracellular matrix on the outer surface. However, in a case where the cell culture does not have an extracellular matrix sufficient to bind the cell culture to the administration site, for example, in a case where single cells are used as the cell culture, the cell culture may be administered in combination with an extracellular matrix in an amount to be required. For example, a sheet-shaped cell culture prepared from 7 to 10 × 10⁵ cells has an extracellular matrix sufficient to bind to the administration site, and an extracellular matrix in an amount corresponding to such a sufficient amount may be administered in combination with a cell culture. In this regard, the expression "single cells" refers to cells that do not adhere to each other and are present in a single state, and includes cells obtained by separating multiple cells that have been originally adhered to each other into single cells, and cells prepared in a single-cell state. Further, in the present specification, the single cells include cells having a morphology similar to that of a single cell. In this regard, the expression "morphology similar to that of a single cell" refers to a morphology in which a small number of cells are adhered to each other in at least some of the cells but with which such cells behave in a similar manner to a single cell. The cells in the morphology similar to that of a single cell contain cells in a state of being bound to each other by 1 to 30%, preferably 1 to 20%, and more preferably 1 to 10%.

The cells contained in the fragment of the sheet-shaped cell culture for use in the present invention are not particularly limited as long as they can produce an extracellular matrix, and include myoblast cells.

In one embodiment, as the cells contained in the fragment of the sheet-shaped cell culture for use in the present invention, cells capable of promoting the angiogenesis is mentioned, for example, cells capable of secreting a factor that promotes the angiogenesis, for example, a cytokine such as VEGF are particularly preferred.

The cells forming the fragment of the sheet-shaped cell culture for use in the present invention can be derived from any organism that is to be subject to treatment with the cell culture, and examples of such an organism include, but are not limited to, a human, primates, a dog, a cat, a pig, a horse, a goat, a sheep, rodent animals (such as a mouse, a rat, a hamster, and a guinea pig), and a rabbit. Further, as the cells forming the fragment of the sheet-shaped cell culture for use in the present invention, only one kind of, or two or more kinds of cells may be used. In a preferred embodiment of the present invention, in a case where there are two or more kinds of the cells that form the fragment of the sheet-shaped cell culture for use in the present invention, the content ratio (purity) of the most abundant cells is 60% or more, preferably 70% or more, and more preferably 75% or more, at the end of the cell culture production.

The cells may be cells derived from heterogeneous cells, or may also be cells derived from homogeneous cells. In this regard, in a case where the cell culture is used for transplantation, the expression "cells derived from heterogeneous cells" means cells derived from an organism of a species different from that of the recipient. For example, in a case where the recipient is a human, cells derived from a monkey or a pig correspond to the cells derived from heterogeneous cells. Further, the expression "cells derived from homogeneous cells" means cells derived from an organism of the same species as that of the recipient. For example, in a case where the recipient is a human, human cells correspond to the cells derived from homogeneous cells. The cells derived from homogeneous cells include self-derived cells (also referred to as "self cells" or "autologous cells"), that is, recipient-derived cells, and cells derived from homogeneous non-self cells (also referred to as "non-autologous cells"). The self-derived cells are preferable in the present disclosure because the cells do not cause any rejection even when being transplanted. However, cells derived from heterogeneous cells, or cells derived from homogeneous non-self cells can also be used. In a case where such cells derived from heterogeneous cells or derived from homogeneous non-self cells are used, the cells may require immunosuppressive treatment in order to suppress the rejection, in some cases. Note that in the present specification, the cells other than the self-derived cells, that is, cells derived from heterogeneous cells and cells derived from homogeneous non-self cells may also be collectively referred to as "non-self-derived cells". In one embodiment of the present disclosure, the cells are autologous cells or non-autologous cells. In one embodiment of the present disclosure, the cells are autologous cells. In another embodiment of the present disclosure, the cells are non-autologous cells.

In the present invention, the fragment of the sheet-shaped cell culture for use in the present invention is administered into a tissue of a subject, that is to a lesion site of a subject with disease in the lower limbs. In one embodiment, in a patient with peripheral arterial disease, the fragment of the sheet-shaped cell culture is administered to a lesion site where the blood flow is deteriorated, for example, to the adductor muscle group of the thigh in the lower limbs. In one embodiment, by administering the fragment of the sheet-shaped cell culture to a lesion site, for example, the blood flow can be improved up to, for example, 35%, 40%, or 45% as compared with the healthy state on the 7th day after the administration. In one embodiment, by administering the fragment of the sheet-shaped cell culture to a lesion site, for example, the blood flow can be improved by around 1.5 times, around 2 times, or around 2.5 times as compared with the control saline solution. Preferably, the fragment of the sheet-shaped cell culture is administered into a tissue by injection. Accordingly, the fragment of the sheet-shaped cell culture for use in the present invention has a size enough to pass through the inside of an injection needle to be used for administration, and further enough to prevent the cell culture from entering the microvessel after the administration. As to such a size, a cell culture having an average diameter of, for example, 30 um to 1000 µm, and preferably 100 um to 500 µm can be mentioned. In this regard, when a cell culture is planarly observed under a microscope or the like, a cell culture having an average diameter of 100 µm to 500 µm refers to, for example, a cell culture of which all the line segments connecting between any corners is 100 um to 500 um in a case where the observed shape is polygonal, or a cell culture having major and minor diameters of 100 um to 500 µm, respectively in a case where the observed shape is substantially circular. In one embodiment, examples of the cell culture having such a size include a sheet-shaped cell culture having around 10² to 10⁶ cells, a fragment of a sheet-shaped cell culture, and a spheroid. The fragment of the sheet-shaped cell culture for use in the present invention has a size of 100 to 500 µm.

In the present invention, the expression "disease in the lower limbs" is a disease in which blood vessels are narrowed or occluded in the lower limbs and the blood flow in the lower limbs is deteriorated: The disease is peripheral arterial disease. In the present invention, examples of the peripheral arterial disease include, but are not limited to, arteriosclerosis obliterans, Buerger disease, and collagen disease, and particularly severe arteriosclerosis obliterans is referred to as critical limb ischemia.

Further, the term "treating" in the present invention includes cure of disease, and all types of medically acceptable prophylactic and/or therapeutic interventions for the purpose of temporary remission, prophylaxis or the like. For example, the term "treating" includes medically acceptable intervention for the various purposes, including retardation or suspension of the progression of a disease associated with abnormalities of tissue, regression or disappearance of lesion, prevention of the onset or recurrence of the disease, and the like.

Without being bound by any particular theory, the action of the fragment of the sheet-shaped cell culture of the present invention on peripheral arterial disease is considered because a cytokine such as VEGF, which is persistently secreted from the administered fragment of the sheet-shaped cell culture of the present invention, acts directly and/or indirectly on the cells at an ischemic site to promote the angiogenesis. Further, it is considered that the fragment of the sheet-shaped cell culture that is used in the present invention contains an extracellular matrix, and thus, the survival rate of the cell culture at an ischemic site becomes high, and the action is efficiently performed on the cells at the ischemic site. As described above, it is considered that the fragment of the sheet-shaped cell culture that is used in the present invention has a high survival rate at an ischemic site, and acts on the cells at the ischemic site to promote the angiogenesis, and thus the myogenesis can be generated in the early stage. For example, it is considered that it takes two months for a model of ischemia to recover the muscle (see, Mohiuddin M et. al, Scientific Reports volume 9, Article number: 9551 (2019)), but by using the cell culture of the present invention, for example, the muscle fibers can be generated in 3 days, and the myogenesis can be completed in 1 month.

By administering the fragment of the sheet-shaped cell culture that is used in the present invention to an ischemic site, and allowing the fragment of the sheet-shaped cell culture to act on the cells at the ischemic site, centrally nucleated muscle fibers (newly formed muscle fibers) can be generated. The generation of such muscle fibers can be confirmed, for example, by counting the number of mature muscle fibers having a nucleus in the margin (periphery) and the number of centrally nucleated muscle fibers (newly formed muscle fibers) in respective hematoxylin-eosin stained tissues, by counting the number of embryonic MHC-positive cells in the tissue, or by analyzing the gene expression of an angiogenesis-related factor such as VEGF, HGF, FGF, Ang-1, Ang-2, or SDF-1, and a muscle differentiation regulator such as Pax7, or MyoD. In one embodiment, by administering the cell culture of the present invention to an ischemic site, for example, centrally nucleated muscle fibers can be generated within 2 days, 3 days, or 4 days, and as described above, the cure of the muscle can be confirmed.

In the present invention, the fragment of the sheet-shaped cell culture contains cells derived from skeletal muscle, wherein the cells derived from skeletal muscle are myoblast cells. In such an embodiment, the cells forming the fragment of the sheet-shaped cell culture of the present invention contain the cells derived from skeletal muscle by 50% or more, 60% or more, 65% or more, 70% or more, 75% or more, 80% or more, 85% or more, or 90% or more, and preferably 60% or more.

The myoblast cells are well known in the technical field to which the present invention belongs, and can be prepared from skeletal muscle by any known method (for example, method disclosed in JP 2007-89442 A, or the like), and as the myoblast cells, for example, catalog number: CC-2580 of Lonza Japan, product code 3520 of Cosmo Bio Co., Ltd., or the like can be obtained commercially. The myoblast cells are not limited to such cells, and can be identified by a marker such as CD56, α7 integrin, myosin heavy chain IIa, myosin heavy chain IIb, myosin heavy chain IId (IIx), MyoD, Myf5, Myf6, myogenin, desmin, or PAX3. In one embodiment, the myoblast cells are CD56 positive. In one embodiment, the myoblast cells are CD56 positive and desmin positive.

In a case where the myoblast cells are prepared from striated muscle tissue, the prepared cell population contains fibroblast cells. When the fragment of the sheet-shaped cell culture for use in the present invention is produced, in a case where a cell population containing the myoblast cells prepared from striated muscle tissue is used, a certain amount of fibroblast cells is contained in the cell population. The fibroblast cells are well known in the technical field to which the present invention belongs, and can be identified by a marker such as TE-7 (see, for example, Rosendaal et al., J Cell Sci. 1994, 107(Pt1): 29-37, Goodpaster et al. J Histochem Cytochem. 2008, 56(4): 347-358, and the like).

In one embodiment, the cells forming the fragment of the sheet-shaped cell culture that is used in the present invention include the myoblast cells prepared from striated muscle tissue. Accordingly, the cell population used in production of the fragment of the sheet-shaped cell culture that is used in the present invention can contain myoblast cells and fibroblast cells. In one embodiment, the cell population used in production of the fragment of the sheet-shaped cell culture of the present invention can have a CD56-positive rate of 50% or more, 60% or more, 65% or more, 70% or more, 75% or more, 80% or more, 85% or more, or 90% or more, and preferably 60% or more.

The cell population used in production of the fragment of the sheet-shaped cell culture that is used in the present invention can contain fibroblast cells, and in a case where the content of the fibroblast cells is extremely high, the content of myoblast cells is lowered, and thus, this is not preferable. Accordingly, in one embodiment, the cell population used in production of the fragment of the sheet-shaped cell culture of the present invention can have a TE-7 positive rate of 50% or less, 40% or less, 35% or less, 30% or less, 25% or less, 20% or less, 150 or less, or 10% or less, and preferably 40% or less.

The cell population used in production of the fragment of the sheet-shaped cell culture that is used in the present invention can contain cells other than the myoblast cells and the fibroblast cells, but the smaller the number of such cells is, the more preferable the cell population is. Accordingly, the higher the total value of the CD56-positive rate and the TE-7 positive rate is, the more preferable the cell population is, and the total value can be, for example, 80% or more, 85% or more, 90% or more, 95% or more, 96% or more, 97% or more, 98% or more, or 99% or more, and preferably 90% or more.

In the present invention, a cytokine is secreted from a fragment of the sheet-shaped cell culture that is used in the present invention containing cells derived from skeletal muscle.

In the present invention, examples of the cytokine secreted from a fragment of a sheet-shaped cell culture that is used in the present invention containing cells derived from skeletal muscle include, but are not limited to, IL-1b, IL-1ra, IL-2, IL-4, IL-5, IL-6, IL-7, IL-8, IL-9, IL-10, IL-12, IL-13, IL-15, IL-17, eotaxin, FGF-basic, G-CSF, GM-CSF, IFN-g, IP-10, MCP-1 (MCAF), MIP-1a, PDGF-bb, MIP-1b, RANTES, TNF-a, HGF-1, SDF-1, and VEGF.

The cell culture that is used in the present invention is a fragment of a sheet-shaped cell culture. In the present invention, the expression "fragment of a sheet-shaped cell culture" refers to one obtained by crushing a sheet-shaped cell culture to divide into multiple fragments. The size of the fragment is a size with which most of the fragment does not enter the microvessel, and passes through the inside of an injection needle. As such a size, the average length of diagonal lines of the sheet-shaped plane is 100 um to 500 um. In the present invention, the crushing can be performed by any known method as long as a fragment of a sheet-shaped cell culture can be obtained, for example, the crushing can be performed by suspending a liquid containing a sheet-shaped cell culture with a syringe and a needle, a pipette, or the like.

In the present invention, the expression "sheet-shaped cell culture" means one in a sheet shape formed by connecting cells to each other. The cells may be connected to each other directly (including connection through a cellular element such as an adhesion molecule) and/or through a mediator. The mediator is not particularly limited as long as it is a substance that can at least physically (mechanically) connect cells to each other, and as the mediator, for example, an extracellular matrix or the like can be mentioned. The mediator is preferably derived from a cell, and particularly derived from a cell that forms a cell culture. The cells are at least physically (mechanically) connected to each other, but may be more functionally, for example, chemically electrically connected to each other. The sheet-shaped cell culture may be formed of one cell layer (single layer), or may also be formed of two or more cell layers (such as a laminated (multilayer) body, for example, two-layer, three-layer, four-layer, five-layer, or six-layer). Further, the sheet-shaped cell culture may have a three-dimensional structure having a thickness exceeding the thickness of one cell without showing any clear layered structure of the cells. For example, in the vertical section of the sheet-shaped cell culture, the cells may be present in a state of being non-uniformly (for example, mosaic-like) arranged without being uniformly aligned in the horizontal direction.

The sheet-shaped cell culture preferably does not contain a scaffold (support). The scaffold may be used in some cases in the technical field to which the invention belongs in order to attach cells onto the surface of and/or to the inside of the scaffold and to maintain the physical integrity of the sheet-shaped cell culture, and as the scaffold, for example, a membrane made of polyvinylidene difluoride (PVDF) or the like is known, but the sheet-shaped cell culture of the present disclosure can maintain the physical integrity even without such a scaffold. Further, the sheet-shaped cell culture of the present disclosure preferably consists only of cell-derived substances forming the sheet-shaped cell culture, and does not contain any other substances.

In the present invention, the thickness of the sheet-shaped cell culture is not particularly limited. In a case where a single-layer sheet is used as the sheet-shaped cell culture, the thickness is usually a thickness of one or more cells, and varies depending on the kind of the cells forming the sheet-shaped cell culture, and in one embodiment, the sheet-shaped cell culture of the present invention has a thickness of 30 um or more, and in one preferred embodiment, the sheet-shaped cell culture has a thickness of 50 um or more. Examples of the range of the value of the sheet-shaped cell culture of the present invention include 30 um to 200 um, preferably 50 um to 150 µm, and more preferably 60 um to 100 um. In a case where a laminated sheet is used as the sheet-shaped cell culture, the thickness does not exceed the value obtained by a thickness of the single-layer sheet × the number of laminated sheets. Accordingly, as one embodiment, in a case where, for example, a sheet obtained by stacking five single-layer sheets in layers is used, the sheet has a thickness of 150 um or more, and in one preferred embodiment, the sheet has a thickness of 250 um or more. In that case, examples of the range of the value of the sheet-shaped cell culture of the present invention include 150 um to 1000 um, preferably 250 um to 750 µm, and more preferably 300 µm to 500 µm.

Another aspect of the present invention relates to a method for preparing a fragment of a sheet-shaped cell culture that is used in the present invention, including: a step of seeding cells on a substrate; a step of forming the seeded cells into a sheet-shaped cell culture; and a step of crushing the formed sheet-shaped cell culture.

In the preparation method of a fragment of a sheet-shaped cell culture that is used in the present invention, the sheet-shaped cell culture can be produced by any method known to a person skilled in the art (see, for example, Patent Literature 1, JP 2010-081829 A, JP 2011-110368 A, and the like). The method for producing a sheet-shaped cell culture typically includes, but is not limited to, a step of seeding cells on a substrate, a step of forming the seeded cells into a sheet-shaped cell culture, and a step of detaching the formed sheet-shaped cell culture from the substrate. In this regard, in the step of forming the seeded cells into a sheet-shaped cell culture, a layer of extracellular matrix is formed on one surface on the substrate side of the sheet-shaped cell culture, and in the subsequent step of detaching the formed sheet-shaped cell culture from the substrate, the sheet-shaped cell culture is detached from the substrate while maintaining the layer of extracellular matrix on the one surface. Before the step of seeding cells on a substrate, a step of freezing cells and a step of thawing the cells may be performed. Further, a step of washing the cells may be performed after the step of thawing the cells. Each of these steps can be performed by any known technique suitable for the production of a sheet-shaped cell culture. The step of producing a sheet-shaped cell culture may include 1 or 2 or more of the steps according to the above method for producing a sheet-shaped cell culture as the sub-steps. In one embodiment, a step of proliferating the cells is not included after the step of thawing the cells and before the step of seeding the cells on a substrate.

The preparation method of a fragment of a sheet-shaped cell culture that is used in the present invention further includes a step of crushing a sheet-shaped cell culture formed after production of the sheet-shaped cell culture. The step of crushing the sheet-shaped cell culture can be performed by any known technique as long as the sheet-shaped cell culture is separated into multiple cell cultures, and a fragment of the sheet-shaped cell culture can be prepared. In one embodiment, the crushing of the sheet-shaped cell culture is to repeat a set of suction and discharge of the sheet-shaped cell culture 2 to 8 times by using a syringe and a needle and to suspend, and is preferably to repeat the set of suction and discharge 4 to 6 times and to suspend.

Examples of the substrate are not particularly limited as long as cells can form a cell culture on the substrate, and include containers made of various materials, and a solid or half-solid surface in a container. It is preferable that the container has a structure/material that does not allow a liquid such as a liquid culture medium to permeate. Examples of the material include, but are not limited to, polyethylene, polypropylene, Teflon (registered trademark), polyethylene terephthalate, polymethyl methacrylate, nylon-6,6, polyvinyl alcohol, cellulose, silicon, polystyrene, glass, polyacrylamide, polydimethyl acrylamide, and a metal (for example, iron, stainless steel, aluminum, copper, or brass). Further, it is preferable that the container has at least one flat surface. As such a container, without any limitation, for example, a culture container provided with the bottom made of a substrate capable of forming a cell culture and the liquid-impermeable side can be mentioned. Specific examples of the culture container include, but are not limited to, a cell-culture dish, and a cell-culture bottle. The bottom of the container may be transparent or opaque. If the bottom of a container is transparent, cells can be observed and counted from the underside of the container. Further, the container may have a solid or half-solid surface inside thereof. Examples of the solid surface include a plate, and a container, which are made of various materials as described above, and examples of the half-solid surface include a gel, and a soft polymer matrix. As the substrate, a substrate prepared by using the above-described materials may be used, or a commercially available material may be used. As the preferred substrate, without any limitation, for example, a substrate having an adhesive surface, which is suitable for forming a sheet-shaped cell culture, can be mentioned. Specifically, a substrate having a hydrophilic surface, for example, a substrate of which the surface is coated with a hydrophilic compound such as corona discharge-treated polystyrene, a collagen gel, or a hydrophilic polymer, a substrate of which the surface is coated with an extracellular matrix of collagen, fibronectin, laminin, vitronectin, proteoglycan, glycosaminoglycan, or the like, or a cell adhesion factor such as a cadherin family, a selectin family, or an integrin family, or the like can be mentioned. Further, such a substrate is commercially available (for example, Corning (registered trademark) TC-Treated Culture Dish, Corning, or the like). The overall or part of the substrate may be transparent or opaque.

The surface of the substrate may be coated with a material of which the physical properties change in response to a stimulus, for example, temperature or light. As such a material, without any limitation, a known material, for example, a temperature-responsive material made of a homopolymer or a copolymer of a (meth)acrylamide compound, a N-alkyl-substituted (meth)acrylamide derivative (for example, N-ethyl acrylamide, N-n-propyl acrylamide, N-n-propyl methacrylamide, N-isopropyl acrylamide, N-isopropyl methacrylamide, N-cyclopropyl acrylamide, N-cyclopropyl methacrylamide, N-ethoxyethyl acrylamide, N-ethoxyethyl methacrylamide, N-tetrahydrofurfuryl acrylamide, N-tetrahydrofurfuryl methacrylamide, or the like), a N,N-dialkyl-substituted (meth)acrylamide derivative (for example, N,N-dimethyl (meth)acrylamide, N,N-ethyl methyl acrylamide, N,N-diethyl acrylamide, or the like), a (meth)acrylamide derivative having a cyclic group (for example, 1-(1-oxo-2-propenyl)-pyrrolidine, 1-(1-oxo-2-propenyl)-piperidine, 4-(1-oxo-2-propenyl)-morpholine, 1-(1-oxo-2-methyl-2-propenyl)-pyrrolidine, 1-(1-oxo-2-methyl-2-propenyl)-piperidine, 4-(1-oxo-2-methyl-2-propenyl)-morpholine, or the like), or a vinyl ether derivative (for example, methyl vinyl ether), or a photoresponsive material such as a light-absorbing polymer having an azobenzene group, a copolymer of a vinyl derivative of triphenylmethane leucohydroxide and an acrylamide-based monomer, or N-isopropylacrylamide gel containing spirobenzopyran, may be used (see, for example, JP H02-211865 A, and JP 2003-33177 A). By giving a predetermined stimulus to such a material, the physical properties, for example, the hydrophilicity and the hydrophobicity can be changed, and the detachment of a cell culture attached on the material can be promoted. A culture dish coated with a temperature-responsive material is commercially available (for example, UpCell (registered trademark) of CellSeed Inc., or Cepallet (registered trademark) of DIC Corporation), and such a culture dish can be used in the production method of the present disclosure.

The substrate may have various shapes, but is preferably flat. Further, the area is not particularly limited, and may be, for example, around 1 cm² to around 200 cm², around 2 cm² to around 100 cm², or around 3 cm² to around 50 cm². For example, as the substrate, a circular culture dish having a diameter of 10 cm can be mentioned. In this case, the area is 56.7 cm².

The substrate may be coated with serum. By using a substrate coated with serum, a sheet-shaped cell culture with a higher density can be formed. The expression "coated with serum" means a state that a serum component adheres onto a surface of the substrate. Such a state can be obtained, for example, by processing a substrate with serum, without any limitation. The processing with serum includes bringing serum into contact with a substrate, and performing the incubation for a predetermined period of time as needed.

As the serum, heterologous serum and/or homologous serum can be used. In a case where a sheet-shaped cell culture is used for transplantation, the heterologous serum means serum derived from an organism of a species different from that of the recipient. For example, in a case where the recipient is a human, serum derived from a bovine or a horse, for example, fetal bovine serum/fetal calf serum (FBS/FCS), calf serum (CS), horse serum (HS), or the like corresponds to the heterologous serum. Further, the expression "homologous serum" means serum derived from an organism of the same species as that of the recipient. For example, in a case where the recipient is a human, human serum corresponds to the homologous serum. The homologous serum includes self serum (also referred to as "autologous serum"), that is, serum derived from the recipient, and homologous non-autologous serum derived from an individual of the same species other than the recipient. Note that in the present specification, serum other than self serum, that is, heterologous serum and homologous non-autologous serum may be collectively referred to as "non-self serum".

The serum for coating on a substrate is commercially available, or can be prepared from the blood collected from a desired organism by a conventional method. Specifically, for example, a method in which the collected blood is left to stand at room temperature for around 20 minutes to around 60 minutes so as to be coagulated, the coagulated blood is centrifuged at around 1000×g to around 1200×g, and a supernatant is collected, or the like can be mentioned.

In a case where the incubation is performed on a substrate, serum may be used in undiluted form, or may be diluted for use. The serum can be diluted, without any limitation, in any medium, for example, water, a saline solution, various buffer solutions (for example, PBS, HBSS and the like), various liquid media (for example, DMEM, MEM, F12, DMEM/F12, DME, RPMI1640, MCDB (such as MCDB102, 104, 107, 120, 131, 153, or 199), L15, SkBM, or RITC80-7) or the like. The dilution concentration is not particularly limited as long as the serum component can adhere onto a substrate, and is, for example, around 0.5% to around 1000 (v/v), preferably around 1% to around 60% (v/v), and more preferably around 5% to around 40% (v/v).

The incubation time is also not particularly limited as long as the serum component can adhere onto a substrate, and is, for example, around 1 hour to around 72 hours, preferably around 2 hours to around 48 hours, more preferably around 2 hours to around for 24 hours, and furthermore preferably around 2 hours to around 12 hours. The incubation temperature is also not particularly limited as long as the serum component can adhere onto a substrate, and is, for example, around 0°C to around 60°C, preferably around 4°C to around 45°C, and more preferably room temperature to around 40°C.

The serum may be discarded after incubation. As the technique for discarding the serum, suction with a pipette or the like, or a conventionally-used technique for discarding liquid such as decantation can be used. In a preferred embodiment of the present disclosure, after the serum is discarded, the substrate may be washed with a serum-free washing solution. The serum-free washing solution is not particularly limited as long as it is a liquid medium that does not contain serum and does not adversely affect the serum component adhered onto a substrate, and the washing can be performed, without any limitation, for example, by water, a saline solution, various buffer solutions (for example, PBS, HBSS and the like), various liquid media (for example, DMEM, MEM, F12, DMEM/F12, DME, RPMI1640, MCDB (such as MCDB102, 104, 107, 120, 131, 153, or 199), L15, SkBM, or RITC80-7), or the like. As the washing technique, without any limitation, a conventionally-used technique for washing a substrate, for example, a technique in which a serum-free washing solution is added onto a substrate, stirred for a predetermined time (for example, around 5 seconds to around 60 seconds), and then discarded, or the like can be used.

In the present disclosure, the substrate may be coated with a growth factor. In this regard, the expression "growth factor" means any substance that promotes proliferation of cells as compared with a substrate that is not coated with the growth factor, and examples of the growth factor include epidermal growth factor (EGF), vascular endothelial growth factor (VEGF), and fibroblast growth factor (FGF). The technique for coating on a substrate with a growth factor, the discarding technique, and the washing technique are basically the same as those of serum except that the dilution concentration at the time of incubation is, for example, around 0.000 1 ug/mL to around 1 µg/mL, preferably around 0.0005 µg/mL to around 0.05 µg/mL, and more preferably around 0.001 µg/mL to around 0.01 µg/mL.

In the present disclosure, the substrate may be coated with a steroid drug. In this regard, the expression "steroid drug" refers to a compound that does not exert an adverse effect on the living body, such as adrenocortical insufficiency, or Cushing syndrome, among the compounds having a steroid nucleus. Examples of the compound include, but are not limited to, cortisol, prednisolone, triamcinolone, dexamethasone, and betamethasone. The technique for coating on a substrate with a steroid drug, the discarding technique, and the washing technique are basically the same as those of serum except that the dilution concentration at the time of incubation is, for example, 0.1 ug/mL to around 100 µg/mL, preferably around 0.4 ug/mL to around 40 µg/mL, and more preferably around 1 ug/mL to around 10 µg/mL, as dexamethasone.

The substrate may be coated with any one of the serum, the growth factor, and the steroid drug, or may be coated with any combination of the serum, the growth factor, and the steroid drug, that is, a combination of the serum and the growth factor, a combination of the serum and the steroid drug, a combination of the serum, the growth factor, and the steroid drug, or a combination of the growth factor and the steroid drug. In a case of coating with multiple components, the coating with these components may be performed at the same time by mixing the components with each other, or may be performed in separate steps.

The substrate may be seeded with cells immediately after being coated with serum and the like, or may also be stored after being coated and then seeded with cells. The coated substrate can be stored for a long period of time, for example, by keeping the substrate at around 4°C or less, preferably around -20°C or less, and more preferably around -80°C or less.

Seeding of cells on a substrate can be performed by any known technique under any known conditions. The seeding of cells on a substrate may be performed, for example, by injecting a cell suspension in which cells are suspended in a liquid culture medium into a substrate (culture container). For the injection of the cell suspension, an instrument suitable for the injection operation of the cell suspension, such as a dropper or a pipette, can be used.

In one embodiment, the seeding can be performed at a density of around 7.1 × 10⁵ cells/cm² to around 3.0 × 10⁶ cells/cm², around 7.3 × 10⁵ cells/cm² to around 2.8 × 10⁶ cells/cm², around 7.5 × 10⁵ cells/cm² to around 2.5 × 10⁶ cells/cm², around 7.8 × 10⁵ cells/cm² to around 2.3 × 10⁶ cells/cm², around 8.0 × 10⁵ cells/cm² to around 2.0 × 10⁶ cells/cm², around 8.5 × 10⁵ cells/cm² to around 1.8 × 10⁶ cells/cm², around 9.0 × 10⁵ cells/cm² to around 1.6 × 10⁶ cells/cm², around 1.0 × 10⁶ cells/cm² to around 1.6 × 10⁶ cells/cm², or the like.

Further, another aspect of the present invention relates to a pharmaceutical composition for use in treating peripheral arterial disease, including a fragment of a sheet-shaped cell culture containing myoblast cells, and an extracellular matrix, wherein the fragment of a sheet-shaped cell culture is as described herein.

The pharmaceutical composition for use in the present invention may contain various additional components such as an extracellular matrix and the like, for example, a pharmaceutically acceptable carrier, a component that enhances the survivability, bioadhesiveness, function, and/or the like of a cell culture, other active components useful in treatment of target disease, and the like, in addition to the cell culture of the present invention. As such additional components, any known additional components can be used, and a person skilled in the art is well-versed in the additional components. Further, the pharmaceutical composition for use in the present invention can be used in combination with a component that enhances the survivability, bioadhesiveness, function, and/or the like of a cell culture, other active components useful in treatment of target disease, and the like. In one embodiment, the pharmaceutical composition for use in the present invention is for use in treatment of disease in the lower limbs. The tissue and disease to be treated are as described above for the fragment of a sheet-shaped cell culture for use in the present invention.

In the pharmaceutical composition for use in the present invention, the cell culture and the extracellular matrix may be separately prepared, and administered at the same time. In the pharmaceutical composition for use in the present invention, as the cell culture, the above-described fragment of a sheet-shaped cell culture is used. In this regard, in a case where a fragment of a sheet-shaped cell culture is administered, an extracellular matrix in an amount sufficient to bind to an ischemic site is formed on an adhesive surface with a substrate when the sheet-shaped cell culture is prepared, and thus, it is not separately required to administer such an extracellular matrix. In a case where a cell culture being a spheroid is administered, it is only required to administer an extracellular matrix in an amount compensating for an amount insufficient relative to the amount of an extracellular matrix contained in the sheet-shaped cell culture formed of the same number of cells, and for example, in a case where a spheroid cell culture has half the amount of the extracellular matrix of a sheet-shaped cell culture, an extracellular matrix in an amount compensating for the other half may be administered. The extracellular matrix is commercially available (for example, VitroCol (trademark) Type I human-derived collagen, or the like of Advanced BioMatrix), and such a commercially available extracellular matrix can be used for the pharmaceutical composition of the present invention.

Also described herein is a method for treating peripheral arterial disease in a subject, including a step of administering an effective amount of the cell culture or pharmaceutical composition of the present invention to a subject in need thereof. The tissue and disease to be subjected to the treatment method of the present invention are as described above for the cell culture of the present invention. Further, in the treatment method described herein, a component that enhances the survivability, bioadhesiveness, function, and/or the like of a cell culture, other active components useful in treatment of target disease, and the like, can be used in combination with the cell culture or pharmaceutical composition of the present invention.

In the present invention, the term "subject" means any individual organism, preferably an animal, more preferably a mammal, and furthermore preferably an individual human.

Further, the term "treatment" includes cure of disease, and all types of medically acceptable prophylactic and/or therapeutic interventions for the purpose of temporary remission, prophylaxis or the like. For example, the term "treatment" includes medically acceptable intervention for the various purposes, including retardation or suspension of the progression of disease, regression or disappearance of lesion, prevention of the onset or recurrence of the disease, and the like.

In the present invention, the term "effective amount" means, for example, an amount (for example, the number of cells contained in a cell culture, the size, weight, or the like of a cell culture) with which the onset or recurrence of disease can be suppressed, the symptoms can be alleviated, or the progression can be retarded or suspended, and preferably an amount with which the onset or recurrence of disease is prevented, or the disease is cured. Further, an amount with which any adverse effect exceeding the benefit of administration is not exerted is preferable. Such an amount can be appropriately determined by, for example, a test or the like in an experimental animal or a disease model animal such as a mouse, a rat, a dog, or a pig, and such a test method is well known to a person skilled in the art. In addition, the size of the histological lesion to be treated can be an important indicator for determining the effective amount. In one embodiment, a total of 7 to 10 × 10⁵ cells can be administered to a mouse disease model with peripheral arterial disease in a cell culture morphology containing around 10² to 10⁶ cells per cell culture. In one embodiment, a total of 7 to 10 × 10⁶ cells can be administered to a human with peripheral arterial disease in a cell culture morphology containing around 10² to 10⁶ cells per cell culture.

Further, described herein is a method for treating peripheral arterial disease, including administering a fragment of a sheet-shaped cell culture, which contains myoblast cells and has an extracellular matrix, or a pharmaceutical composition, which contains a cell culture containing myoblast cells, and an extracellular matrix.

In accordance with the production method of the present invention, the treatment method described herein may further include a step of producing a fragment of the sheet-shaped cell culture of the present invention. Before the step of producing a fragment of the sheet-shaped cell culture, the treatment method of the present invention may further include a step of collecting cells for producing a fragment of a sheet-shaped cell culture from a subject (for example, skin cells, blood cells, or the like in a case of using iPS cells), or a tissue that is a supply source of cells (for example, skin tissue, blood, or the like in a case of using iPS cells). In one embodiment, a subject from which the cells or the tissue to be a supply source of cells is collected is the same individual as a subject to which a fragment of a sheet-shaped cell culture, a pharmaceutical composition, or the like is administered. In another embodiment, a subject from which the cells or the tissue to be a supply source of cells is collected is a different individual in the same species as a subject to which a fragment of a sheet-shaped cell culture, a pharmaceutical composition, or the like is administered. In another embodiment, a subject from which the cells or the tissue to be a supply source of cells is collected is an individual in different species of a subject to which a fragment of a sheet-shaped cell culture, a pharmaceutical composition, or the like is administered.

As the administration method, typically, direct application to the tissue can be mentioned, and preferably, direct application to the muscle tissue of a subject to be administered can be mentioned. In a case of using a fragment of a sheet-shaped cell culture, the fragment of the sheet-shaped cell culture for use in the present invention and pharmaceutical composition for use in the present invention may be administered by various routes through which administration by injection can be performed, for example, by a route such as an intravenous route, an intramuscular route, a subcutaneous route, a topical route, an intraarterial route, an intraportal route, an intraventricular route, or an intraperitoneal route. In one embodiment, the administration method is intramuscular injection to a subject to be administered. The injection may be divided and performed to multiple points in one treatment.

The frequency of administration is typically once per treatment, but in a case where the desired effect cannot be obtained, multiple times of administration are possible. For example, in a case of administering a cell culture having an extracellular matrix on an outer surface of the cell culture, the cell culture may be divided and injected to multiple places or to the same point multiple times. In a case of administering single cells and an extracellular matrix, the single cells and the extracellular matrix may be divided and injected separately once or multiple times, or a mixture of the single cells and the extracellular matrix may be injected once or multiple times to the same point or to multiple places.

### Examples

Example 1: Preparation of fragment of mouse-derived myoblast cell sheet

### (1) Preparation of mouse-derived myoblast cell sheet

A skeletal muscle was collected from the lower limb of a 4-week old C57BL/6 mouse (CLEA Japan, Inc.), processed with a solution containing collagenase and trypsin, and dispersed in single cells. Such a cell was cultured in a 20% FBS-containing MCDB131 medium under the conditions of 37°C and 5% CO₂ until the confluent was obtained, and the cells were collected.

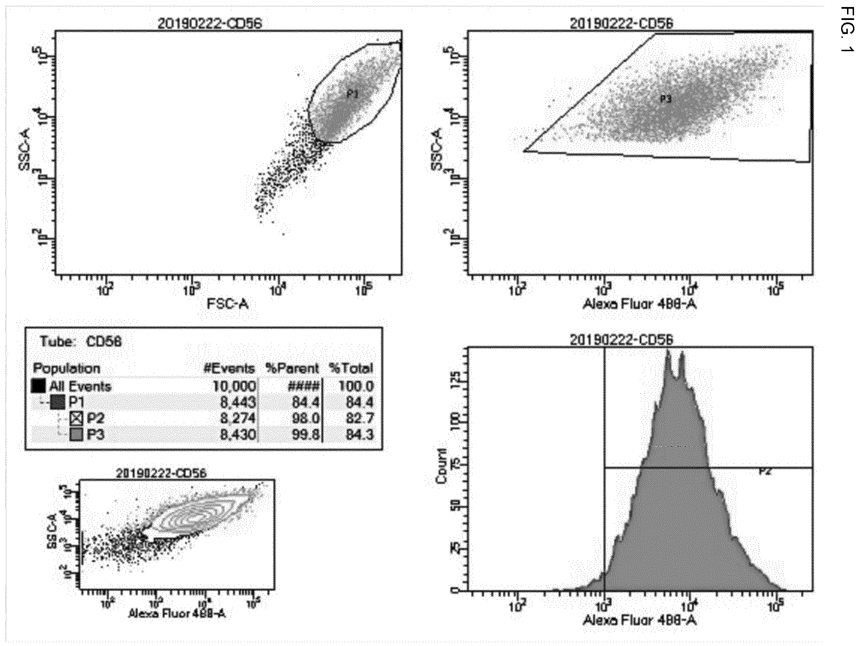

The collected cells were measured by using FACS. The measurement results are shown in Fig. 1. Among the collected cells, 95% or more of the cells were myoblast cells.

The collected cells were seeded at a concentration of 7 to 10 × 10⁵/cm² in a 12-well temperature-responsive culture container (UpCell (registered trademark), 12-multiwell, CS3003, CellSeed Inc.), and cultured in a 20% FBS-containing DMEM/F12 medium for 6 hours or more, and the sheet-forming was performed. After that, by lowering the temperature to 20°C, a sheet-shaped cell culture was detached from the temperature-responsive culture container.

The detached sheet-shaped cell culture was stained with hematoxylin and eosin (HE) to stain the cell nucleus bluish purple with hematoxylin and the other structures red in various densities with eosin, and the cross section was observed with a microscope (20 times). Further, another detached sheet-shaped cell culture was immunostained to stain the cell nucleus blue and the desmin green, and the cross section was observed with a microscope (20 times).

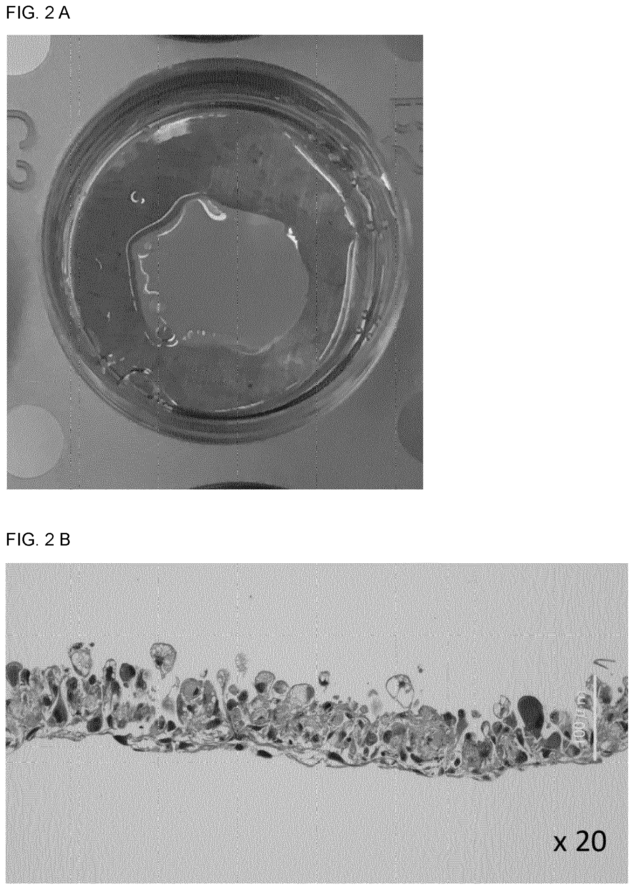

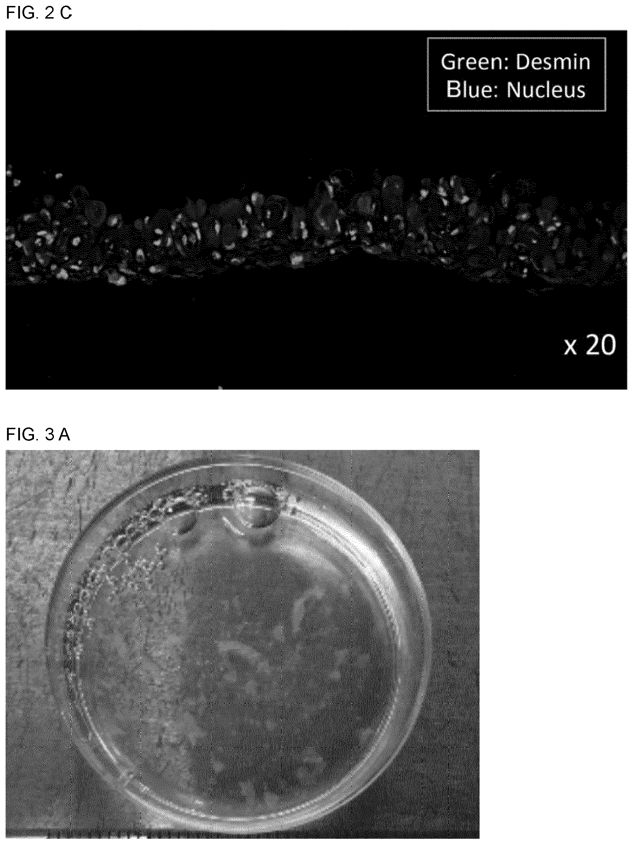

Fig. 2A shows a photograph of the sheet-shaped cell culture, Fig. 2B shows a photomicrograph of the cross section of the hematoxylin-eosin stained sheet-shaped cell culture, and Fig. 2C shows a photomicrograph of the cross section of the immunostained sheet-shaped cell culture.

### (2) Preparation of fragment

Regarding the sheet-shaped cell culture that was not subjected to staining in (1), a culture medium and the like were removed from a temperature-responsive culture container, and 0.3 mL of saline solution was added. After the addition, by using a 1.0-mL syringe (manufactured by TERUMO CORPORATION) and a 23G needle (manufactured by TERUMO CORPORATION), the saline solution was repeatedly sucked and discharged together with the sheet-shaped cell culture to crush the sheet-shaped cell culture, and the crushed sheet-shaped cell culture was suspended in a saline solution. As to a suspension, suspensions prepared by repeating a set of suction and discharge 5 times and 10 times, respectively were prepared.

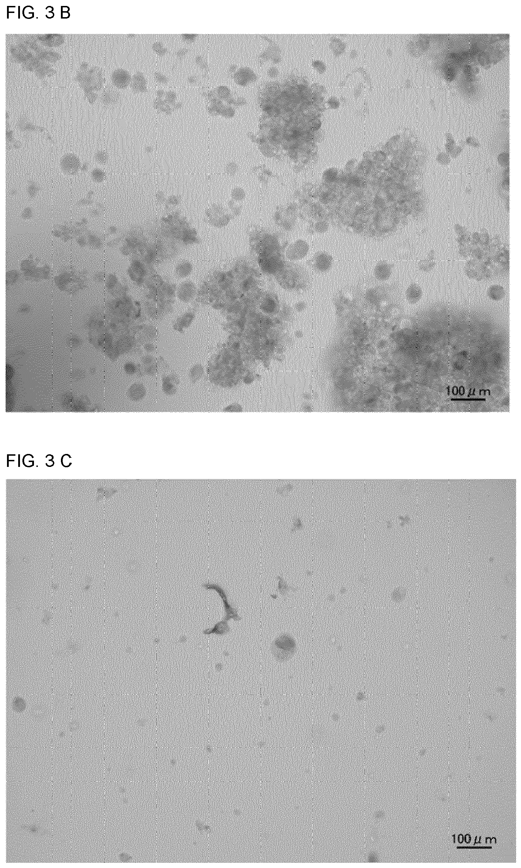

Fig. 3 shows photomicrographs of the respective suspensions. Fig. 3A is a photograph of a suspension prepared by repeating a set of suction and discharge 5 times, and Fig. 3B shows an enlarged photomicrograph of Fig. 3A. Fig. 3C is an enlarged photomicrograph of a suspension prepared by repeating a set of suction and discharge 5 times.

In the suspension prepared by repeating a set of suction and discharge 5 times, a fragment of around 300 to 500 um was confirmed. However, in the suspension prepared by repeating a set of suction and discharge 10 times, almost all the cells were in a morphology close to that of single cells.

The crushed product obtained in Example 1 is considered to be a crushed product having a large amount of extracellular matrix (ECM) present on a surface where a sheet-shaped cell culture binds to a culture substrate.

Example 2: Administration of fragment of sheet-shaped cell culture in mouse model of ischemia

### (1) Preparation of mouse model of ischemia

Seven days before the start of the administration test, ligation and vascular resection were performed beyond the femoral artery in the right hind limb of an 8-week old C57BL/6 mouse (CLEA Japan, Inc.). Before performing the ligation and vascular resection, the blood flow in a mouse in a healthy state was measured by laser Doppler perfusion imaging (LDPI).

### (2) Transplantation

The mouse models of ischemia prepared in (1) were randomly divided into three groups so that each group had 10 mice. The mice in the first group were injected with the suspension prepared by repeating a set of suction and discharge 5 times in Example 1 (hereinafter, referred to as "fragment administration group"). As the control, the mice in the second group were injected with a suspension in which 7 to 10 × 10⁵ cells derived from the same cells as those used for a sheet-shaped cell culture were suspended in 0.3 mL of saline solution in a single-cell form without performing culture (hereinafter, referred to as "single cell administration group"), and the mice in the third group were injected with only 0.3 mL of saline solution (hereinafter, referred to as "saline administration group"). In any mouse in any group, the injection was performed in two divided portions at two places in the muscle of a part where blood vessels of the ischemic limb of a mouse model of ischemia had been removed.

### (3) Test plan

The number of days was counted from the start date of the test by setting the day of injection as day 0, and the test was carried out up to day 28. The blood flow of each mouse was measured by LDPI on days 0, 7, 14, 21, and 28. Further, the tissue at the site of administration was collected on days 1, 3, 5, 7, and 28 in order to confirm the gene expression. The gene expression in the tissue collected from a mouse in each group was evaluated by RT-PCR.

### (4) Results

Results by LDPI of the blood flows % on days 0, 7, 14, 21, and 28 of a mouse in each group were shown in Fig. 4 when the blood flow before the preparation of the mouse model of ischemia was set as 100%. Further, imaging results by LDPI of a representative mouse in each group on days 7, 14, 21 and 28 were shown in Fig. 5. In both of Figs. 4 and 5, the expression "Clustered cells" indicates a mouse in the fragment administration group, the expression "Single cells" indicates a mouse in the single cell administration group, and the expression "Saline" indicates a mouse in the saline administration group. Further, in Fig. 4, the symbol "*" represents that Dunnett's test was performed on the saline administration group and P < 0.05 was obtained.

As shown in Fig. 4, in all the groups, the blood flow on day 0 decreased to less than 20% of that at the preparation time of the mouse model of ischemia, but on days 7, 14, 21, and 28, significant improvement in the blood flow was observed in a mouse in the fragment administration group, as compared with a mouse in the saline administration group and a mouse in the single cell administration group.

As shown in Fig. 5, in all the measurements on days 7, 14, 21, and 28, the blood flow of the mouse in the fragment administration group was improved as compared with the saline administration group and the single cell administration group, and it was confirmed that the blood flow was in a state close to that in the left limb in a healthy state.

As shown in Fig. 6, it was confirmed that VEGF, HGF, FGF, Ang-1, Ang-2, SDF-1, Pax7, and MyoD were expressed at extremely high levels in the tissue collected from a mouse in the fragment administration group as compared with the tissue collected from a mouse in the single cell administration group and the tissue collected from a mouse in the saline administration group.

### Example 3: Confirmation of bioadhesiveness of administered cells

### (1) Test plan

A skeletal muscle was collected from the lower limb of a 4-week old GFP-transduced C57BL/6 mouse (Japan SLC, Inc.), a saline solution was repeatedly sucked and discharged together with a sheet-shaped cell culture in a similar manner to Example 1 to crush the sheet-shaped cell culture, and the crushed sheet-shaped cell culture was suspended in a saline solution to prepare a suspension. Next, mouse models of ischemia were prepared in a similar manner to (1) of Example 2, and the prepared mouse models were divided into three groups. The mice in the first group were injected with the prepared suspension (hereinafter, referred to as "fragment administration group 2"). As the control, the mice in the second group were injected with a suspension in which 7 to 10 × 10⁵ cells of the skeletal muscle collected from the lower limb of a GFP-transduced C57BL/6 mouse were suspended in 0.3 mL of saline solution in a single-cell form without performing culture (hereinafter, referred to as "single cell administration group 2"), and the mice in the third group were injected with only 0.3 mL of saline solution (hereinafter, referred to as "saline administration group 2"). In any mouse in any group, the injection was performed in two divided portions at two places in the muscle of a part where blood vessels of the ischemic limb of a mouse model of ischemia had been removed.

The number of days was counted from the start date of the test by setting the day of injection as day 0, and on days 1, 3, 5, 7, and 28, the mice in respective groups were slaughtered, and the adductor muscle was collected. A part of the tissue obtained from the adductor muscle collected from each mouse was fixed with a formalin solution, and then stained with hematoxylin and eosin, and another part was fixed with a paraformaldehyde solution, and then the frozen specimen was prepared. For each hematoxylin-eosin-stained tissue, the numbers of centrally nucleated muscle fibers (newly formed muscle fibers) and mature muscle fibers having a nucleus in the margin (periphery) were counted. For the frozen specimen, the area of GFP was confirmed. In the tissue collected from a mouse in each group on day 3, the number of embryonic MHC-positive cells was further counted.

### (2) Results

Photomicrographs of the tissues collected from respective mice in the fragment administration group 2, the single cell administration group 2, and the saline administration group 2 on days 3, 5, 7, and 28 and stained with hematoxylin and eosin are shown. In the diagram, the expression "Clustered cells" indicates a mouse in the fragment administration group 2, the expression "Single cells" indicates a mouse in the single cell administration group 2, and the expression "Saline" indicates a mouse in the saline administration group 2, in Fig. 7. Further, results of counting the number of centrally nucleated muscle fibers (newly formed muscle fibers) in respective hematoxylin-eosin stained tissues are shown in Fig. 8A, and results of calculating the ratio between the number of centrally nucleated muscle fibers (newly formed muscle fibers) and the number of mature muscle fibers having a nucleus in the margin (periphery) in respective hematoxylin-eosin stained tissues are shown in Fig. 8B. Further, in the diagram, the symbol "*" represents that Dunnett's test was performed on the saline administration group 2 and P < 0.05 was obtained, and the symbol "+" represents that Dunnett's test was performed on the single cell administration group 2 and P < 0.05 was obtained. Results of counting the number of embryonic MHC-positive cells in the tissues collected from respective mice in the fragment administration group 2, the single cell administration group 2, and the saline administration group 2 on day 3 are shown in Fig. 8C.

As shown in Figs. 8A and 8B, on day 28, the myogenesis was almost completed in the mouse in the fragment administration group 2, whereas the myogenesis was continued in the single cell administration group 2 and the saline administration group 2, and thus, it was confirmed that the myogenesis was generated in an early stage in the mouse in the fragment administration group 2. As shown in Fig. 8C, it was confirmed that on day 3, the number of embryonic MHC-positive cells was remarkably large in the tissue collected from the mouse in the fragment administration group 2 as compared with the tissues collected from the mice in the single cell administration group 2 and the saline administration group 2.

Photomicrographs of frozen specimens of the tissues collected from respective mice in the fragment administration group 2 and the single cell administration group 2 on day 7 are shown in Fig. 9.

As shown in Fig. 9, it was confirmed that the area of GFP was larger in the specimen of the tissue collected from the mouse in the fragment administration group 2 as compared with the specimen of the tissue collected from the mouse in the single cell administration group 2.

## Claims

1. A fragment of a sheet-shaped cell culture, wherein the fragment has a size of 100 to 500 um for use in treating disease in the lower limbs, comprising an extracellular matrix on an outer surface of the cell culture, wherein the disease is a disease in which blood vessels are narrowed or occluded in the lower limbs and the blood flow in the lower limbs is deteriorated, wherein the cell culture contains myoblast cells, and wherein the disease in the lower limbs is peripheral arterial disease.

2. The fragment of a sheet-shaped cell culture for use according to claim 1, wherein the fragment of a cell culture promotes angiogenesis.

3. A fragment of a sheet-shaped cell culture, comprising an extracellular matrix on an outer surface of the cell culture, and wherein the cell culture contains myoblast cells, wherein the fragment of the sheet-shaped cell culture has a size of 100 to 500 µm.

4. A method for preparing a fragment of a sheet-shaped cell culture as defined in claim 1 containing myoblasts, comprising the following steps of:
seeding cells on a substrate;
forming the seeded cells into a sheet-shaped cell culture; and
crushing the formed sheet-shaped cell culture.

5. The method according to claim 4, wherein crushing the sheet-shaped cell culture is to suspend the sheet-shaped cell culture 4 to 6 times by using a syringe.

6. A pharmaceutical composition for use in a method of treating peripheral arterial disease, comprising a fragment of a sheet-shaped cell culture containing myoblast cells; and an extracellular matrix, wherein the fragment of the sheet-shaped cell culture has a size of 100 to 500 um, and wherein the method comprises: administering the pharmaceutical composition.

7. The pharmaceutical composition for use according to claim 6, wherein the administration is intramuscular administration.

## Patentansprüche

1. Fragment einer blattförmigen Zellkultur, wobei das Fragment eine Größe von 100 bis 500 um hat, zur Verwendung bei der Behandlung einer Krankheit in den unteren Gliedmaßen, umfassend eine extrazelluläre Matrix auf einer Außenfläche der Zellkultur, wobei die Krankheit eine Krankheit ist, bei der Blutgefäße in den unteren Gliedmaßen verengt oder verschlossen sind und der Blutfluss in den unteren Gliedmaßen verschlechtert ist, wobei die Zellkultur Myoblastenzellen enthält und wobei die Krankheit in den unteren Gliedmaßen eine periphere arterielle Krankheit ist.

2. Fragment einer blattförmigen Zellkultur zur Verwendung nach Anspruch 1, wobei das Fragment einer Zellkultur die Angiogenese fördert.

3. Fragment einer blattförmigen Zellkultur, umfassend eine extrazelluläre Matrix auf einer Außenfläche der Zellkultur, und wobei die Zellkultur Myoblastenzellen enthält, wobei das Fragment der blattförmigen Zellkultur eine Größe von 100 bis 500 um aufweist.

4. Verfahren zur Herstellung eines Fragments einer blattförmigen Zellkultur wie in Anspruch 1 definiert, enthaltend Myoblasten, umfassend die folgenden Schritte:
Aussäen von Zellen auf ein Substrat;
Bilden der ausgesäten Zellen zu einer blattförmigen Zellkultur; und
Zerkleinern der gebildeten blattförmigen Zellkultur.

5. Verfahren nach Anspruch 4, wobei das Zerkleinern der blattförmigen Zellkultur darin besteht, die blattförmige Zellkultur unter Verwendung einer Spritze 4 bis 6 Mal zu suspendieren.

6. Pharmazeutische Zusammensetzung zur Verwendung in einem Verfahren zur Behandlung einer peripheren arteriellen Krankheit, umfassend ein Fragment einer blattförmigen Zellkultur, die Myoblastenzellen enthält; und eine extrazelluläre Matrix, wobei das Fragment der blattförmigen Zellkultur eine Größe von 100 bis 500 µm aufweist, und wobei das Verfahren umfasst: Verabreichen der pharmazeutischen Zusammensetzung.

7. Pharmazeutische Zusammensetzung zur Verwendung nach Anspruch 6, wobei die Verabreichung eine intramuskuläre Verabreichung ist.

## Revendications

1. Un fragment d'une culture cellulaire en forme de feuille, dans lequel le fragment a une taille de 100 à 500 um pour une utilisation dans le traitement d'une maladie dans les membres inférieurs, comprenant une matrice extracellulaire sur une surface externe de la culture cellulaire, dans lequel la maladie est une maladie dans laquelle les vaisseaux sanguins sont rétrécis ou occluss dans les membres inférieurs et le flux sanguin dans les membres inférieurs est détérioré, dans lequel la culture cellulaire contient des cellules de myoblaste, et dans lequel la maladie dans les membres inférieurs est une maladie artérielle périphérique.

2. Le fragment d'une culture cellulaire en forme de feuille pour une utilisation selon la revendication 1, dans lequel le fragment d'une culture cellulaire favorise l'angiogenèse.

3. Un fragment d'une culture cellulaire en forme de feuille, comprenant une matrice extracellulaire sur une surface externe de la culture cellulaire, et dans lequel la culture cellulaire contient des cellules de myoblaste, dans lequel le fragment de la culture cellulaire en forme de feuille a une taille de 100 à 500 um.

4. Un procédé pour préparer un fragment d'une culture cellulaire en forme de feuille tel que défini dans la revendication 1 contenant des myoblastes, comprenant les étapes suivantes :
ensemencer des cellules sur un substrat ;
former les cellules ensemencées en une culture cellulaire en forme de feuille ; et
écraser la culture cellulaire en forme de feuille formée.

5. Le procédé selon la revendication 4, dans lequel l'écrasement de la culture cellulaire en forme de feuille consiste à mettre la culture cellulaire en forme de feuille en suspension 4 à 6 fois en utilisant une seringue.

6. Une composition pharmaceutique pour une utilisation dans un procédé de traitement d'une maladie artérielle périphérique, comprenant un fragment d'une culture cellulaire en forme de feuille contenant des cellules de myoblaste ; et une matrice extracellulaire, dans lequel le fragment de la culture cellulaire en forme de feuille a une taille de 100 à 500 um, et dans lequel le procédé comprend : administrer la composition pharmaceutique.

7. La composition pharmaceutique pour une utilisation selon la revendication 6, dans laquelle l'administration est une administration intramusculaire.
